# EUROPEAN PATENT SPECIFICATION

(11) **EP 1 558 082 B1**
(45) Date of publication and mention of the grant of the patent: **15.11.2006**
(21) Application number: 03763486.2
(22) Date of filing: 10.07.2003
(51) Int. Cl.: A01N 43/50, C07D 233/82

(54) **BIOCIDAL COMPOSITION CONTAINING HALOGENATED HYDANTOIN AND PARAFFIN WAX**
HALOGENIERTES HYDANTOIN UND PARAFFINWACHS ENTHALTENDE BIOZIDE ZUSAMMENSETZUNG
COMPOSITION BIOCIDE CONTENANT HYDANTOINE HALOGENE ET CIRE PARAFFINIQUE

(30) Priority: 10.07.2002 US 192036
(43) Date of publication of application: 03.08.2005
(73) Proprietor: ALBEMARLE CORPORATION, Baton Rouge Louisiana 70801 (US)
(72) Inventor: HOWART, Jonathan, N., Baton Rouge LA 71816 (US)
(74) Representative: van Heesch, Helmut Werner
(86) International application number: PCT/US2003/022179
(87) International publication number: WO 2004/004462

(56) References cited:
- US-A- 4 535 003
- US-A- 5 565 576
- US-A- 5 756 440

## Description

### Technical Field

This invention relates to novel 1,3-dihalo-5,5-dialkylhydantoin compositions which, by virtue of their physical forms and characteristics, are superlative biocidal water-treating agents and brominating agents.

### Glossary

As used herein the terms "halogen", "halogenated", and "halo" are with reference to bromine or chlorine, or both.

### Background

As is well known, a wide variety of different products in the form of powders or small particles are converted into larger end use forms such as prills, flakes, granules, pills, caplets, tablets, wafers, briquettes, or pucks. In producing such products, it is common to utilize materials known as binders. Such materials, when mixed in suitable proportions with the powder or small particles to be compacted, facilitate the production of materials having desirable physical and mechanical properties. While some binders have relatively broad application to various powdery or small particle sized products, there are a number of instances where the binder can only be used for compaction of certain products and not for others. A principal reason for such limitation is chemical incompatibility as between the binder and certain powdery or small particle sized materials.

One type of material that tends to be difficult to produce in compacted forms such as tablets, granules, and briquettes is halogenated hydantoins, especially N;N'-dihalogenated dialkylhyantoin products such as 1,3-dichloro-5,5-dimethylhydantoin, N,N'-bromochloro-5,5-dimethylhydantoin, and 1,3-dibromo-5,5-dimethylhydantoin. Such materials are useful as biocides for treating water such as recreational water, cooling water, process water, and wastewater.

The N,N'-dihalogenated dialkylhydantoin products are usually formed as powdery solids. For use in many applications such as water treatment, the dry powders need to be converted into larger forms such as granules, tablets, or briquettes. This in turn has presented problems associated with providing densified or compacted products with sufficient strength to withstand the physical stresses encountered in packaging, conveying, handling, shipping, storage, and use. The nature of these problems have been described, for example, in U.S. Pat. Nos. 4,532,330; 4,560,766; 4,654,424; 4,677,130; 4,745,189; and 5,565,576. The approaches described in these patents for alleviating one or more such problems involve use of other materials. Thus in U.S. Pat. Nos. 4,532,330 and 4,621,096, halogenated dimethylhydantoins are mixed with calcium chloride and water, and the mixture is compacted by compression into the desired shape. In U.S. Pat. Nos. 4,560,766 and 4,654,424, halogenated ethylhydantoins are used instead of halogenated dimethylhydantoins and are compacted as such, or are melt blended with halogenated dimethylhydantoins. U.S. Pat. No. 4,677,130 describes forming dry blends of the halogenated dimethylhydantoin with particulate alkali metal or alkaline earth metal salt followed by compression to form a compacted product such as a tablet.

Manufacturers of halogenated hydantoins have sought to overcome these limitations by blending the materials with process additives designed to improve compaction characteristics. The presence of other halogenated hydantoins has also been indicated to provide benefits. For example, published PCT Application WO 97/43264 describes the use of 1,3-bromochloro-5-methyl-5-propyl-hydantoin as a binder in making compacted forms of halogenated hydantoins. The presence of hydantoins having at least one ethyl group in the 5-position is indicated to provide free flowing, dust-free powders which can be compressed into shapes without resorting to binders, as detailed in U.S. Pat. Nos. 4,427,692 and 4,560,766. In U.S. Pat. No. 4,677,130 a series of inorganic salt additives ranging from sodium carbonate to sodium metasilicate was indicated to improve the crush strength of halogenated hydantoin tablets. Inert binders such as fatty acid salts and a hectorite clay were advocated in U.S. Pat. No. 5,756,440, while the use of fatty acid amide binder additives were described in U.S. Pat. No. 5,565,576 and indicated to improve the compaction properties of halogenated hydantoins. U.S. Pat. No. 5,780,641 describes a chemical composition comprising a halogenated hydantoin mixed with dry calcium hydroxide for the purpose of facilitating processing and achieving a shape-retentive form.

Unfortunately, almost all prior efforts in the compaction of halogenated hydantoins have not provided binders having satisfactory compaction characteristics along with good chemical compatibility. Some of the classical binders (*e.g*., polyvinylpyrrolidinone, cellulose compounds, glues, gums, sugars, and starches) which are used to compact other products would react with halogenated hydantoins, in some cases vigorously. Moreover, a number of binder systems proposed for use with halogenated hydantoins do not provide compacted products having sufficient physical and mechanical stability. Low crush strength is often another deficiency of such compacted products.

It can be seen that a need exists for a new type of binder having widespread applicability to powdery and finely-divided substrate materials, especially halogenated hydantoins. It would be of particular advantage if such binders could provide compacted products having superior physical and mechanical properties.

This invention is deemed to fulfill most, if not all, of the foregoing needs.

### Summary of the Invention

Pursuant to this invention, a new type of binding agent for halogenated hydantoins has been discovered. These binders produce compacted compositions of great mechanical and physical strength. Moreover, these binders are strongly hydrophobic, and consequently can be used for modifying the dissolution or release rate of the compacted material in aqueous media. Also, because they are produced and used for other purposes, a number of the materials discovered to be binders pursuant to this invention are available in the marketplace at reasonable cost. Thus, the invention enables the production of compacted compositions such as granules, caplets, tablets, briquettes, pucks, and other shapes with very desirable properties on a highly cost-effective basis.

Thus, in one of its embodiments, this invention provides a shape-retentive compacted composition. The composition comprises a pressure compacted blend of a powdery or finely-divided halogenated dialkylhydantoin and a binder quantity of a paraffin wax. The paraffin wax may be a hydrocarbon paraffin wax and/or a chlorinated paraffin wax. The wax used pursuant to this invention is compatible with halogenated hydantoins, a surprising result, because other waxes are known to be reactive to halogenated hydantoins.

In particular, prior small particle sized product, when released from a tableting die, normally would "delaminate", meaning that the compacted tablet would break apart into smaller pieces. In sharp contrast, 1,3-dihalo-5,5-dimethylhydantoins, especially 1,3-dibromo-5,5-dimethylhydantoin, can be directly converted into tablets of high physical integrity when using a suitable paraffin wax as a binder.

In another of its embodiments, this invention provides a method of producing a shape-retentive compacted composition. The method comprises pressure compacting a blend of a powdery or finely-divided halogenated dialkylhydantoin and a binder quantity of a paraffin wax. The paraffin wax may be a hydrocarbon paraffin wax and/or a chlorinated paraffin wax. Here again, the wax used is compatible with the halogenated hydantoins.

Still another embodiment of this invention involves the provision of dry blends of a powdery or finely-divided halogenated hydantoin and a paraffin wax, compatible with the halogenated dialkylhydantoin. The paraffin wax may be a hydrocarbon paraffin wax and/or a chlorinated paraffin wax. These blends are of particular utility in the manufacture of pressure compacted products formed therefrom. Thus, these dry blends can be produced, stored, and shipped to locations where such compacting operations are to be carried out. Preferably, the amount of the paraffin wax is an amount which is effective to form the compacted product without further addition of either component. However, the proportions can be adjusted at the site of the compaction, if desired.

The amount of the binding agent effective to form the compacted product may vary, depending upon the nature and characteristics of the halogenated hydantoin and the particular paraffin wax being utilized. Thus the dry blends and the compacted products of this invention can contain varying proportions of these essential components. Generally speaking, the amount of the paraffin wax in the dry blends and used in the formation of the pressure compacted products of this invention will fall within the range of 0.5 to 10 wt%, and preferably in the range of one to 5 wt%, based on the total weight of the halogenated hydantoin and the paraffin wax.

The powdery or finely-divided material is an N-halo-5,5-dialkylhydantoin or, more preferably, an N,N'-dihalo-5,5-dialkylhydantoin, materials which heretofore have proven exceedingly difficult to convert into compacted forms. Moreover, even when compacted, such prior compacted forms of the N,N'-dihalo-5,5-dialkylhydantoins were, in most cases, of low strength and of high friability. It has been discovered that when low levels of the aforementioned waxes are mixed with a halogenated hydantoin, the wax acts as binder during pressure compaction to yield a mechanically stable compacted form of increased strength and of low friability. Furthermore, it has been discovered that the waxes are chemically compatible with the halogenated hydantoin.

A feature of this invention is that it is now possible to formulate blends of one or more halogenated hydantoins with one or more novel binders so that compacted products having improved physical and mechanical properties can be produced. Additionally, adjustment of the amount of binder permits adjustments in the rate of dissolution of the halogenated hydantoin. In short, the dissolution characteristics of the product can be tailor-made to suit the needs of the intended usage of the product. For example, it is possible to produce a compacted form with slow dissolution properties that would be desirable in a toilet bowl puck or in a swimming pool formulation. Similarly, products with much more rapid dissolution characteristics can be prepared for use in shock treatment of water for microbiological control.

Other embodiments, features, and advantages of this invention will become still further apparent from the ensuing description and appended claims.

### Further Detailed Description

The exact mechanism by which the paraffin waxes perform the function of producing durable pressure compacted shapes or forms from powdery, finely-divided solids is not known. Without in any way being limited by theory, it may be that the paraffin wax serves in whole or in part as an adhesive or bonding agent, for example by forming, when under the compaction pressure, a film between adjacent particles that bonds the particles together and thus acts like a pressure-activated cement. It is also possible that the paraffin wax serves in whole or in part as a lubricant which, by reducing the coefficient of friction among adjacent particles, enables the particles to come in closer contact with each other during application of compression pressure so that large numbers of interparticulate bonding or fusion sites are created among the adjacent particles. It is also possible that the paraffin wax enables the particles to be more readily distorted under compression pressure so that the particles can more completely bond or fuse together while under such pressure. Indeed, combinations of these and/or other mechanisms may be taking place during the application of the compression pressure to a mixture of the particulate substrate and the paraffin wax.

Therefore it cannot be over-emphasized that this invention is not intended to be limited, should not be interpreted as being limited, and is not to be limited in any way to any mechanism or theory of operation. Thus, for example, while the term "binder" is used herein with reference to the paraffin wax, such term is not intended to limit this invention to any mechanism, theory, or mode of operation; should not be interpreted as limiting this invention to any mechanism, theory, or mode of operation; and does not limit this invention in any way to any mechanism, theory or mode of operation. Rather the term is used to indicate that the paraffin wax somehow or other functions such that when the compression pressure is released, the particles have come together into a durable form or shape that not only can be released from the mold or nip of the compression rolls without physical damage, but that possess the strength and durability to withstand the physical stresses encountered in packaging, conveying, handling, shipping, storage, and use of the compacted article. Exactly how this result actually comes about in a mechanistic or theoretical sense is immaterial to the practice of this invention.

By the term "hydrocarbon paraffin wax" is meant an unsubstituted paraffin wax containing only carbon and hydrogen. Small amounts of non-hydrocarbon impurities may be tolerated, but it is known that halogenated hydantoins react with oxygen-containing paraffin waxes. The term "chloroparaffin wax" refers to chlorinated paraffin wax. Chlorinated paraffin waxes are normally hydrocarbon waxes in which some of the hydrogen atoms have been substituted with chlorine atoms.

A feature of this invention is that paraffin waxes, which are dry solids, do not contain oils. Oils are undesired, particularly for water treatment applications, where the oily residue would float atop the treated water. Further, some waxes contain water, which will dissolve halogenated hydantoins, another undesirable result. Paraffin waxes do not have these drawbacks.

Various paraffin waxes can be used in the practice of this invention. As noted above, these paraffin waxes are typically hydrocarbon paraffin waxes, chlorinated paraffin waxes, or mixtures thereof. For the chlorinated paraffin waxes, the extent of chlorination is not considered critical, so long as the wax is in sold form. Hydrocarbon paraffin waxes are preferred paraffin waxes in the practice of the invention.

The most important requirement for the paraffin wax is that it is a solid at the conditions for blending and compaction (normally room temperature). Liquid waxes yield pastes, an undesired result. Depending on the wax, the melting point may not be sharp; thus, prior to compaction, the wax should not be at the lower end of its melting point range at blending and compaction conditions (usually room temperature). When using two or more waxes, they need not be mixed together prior to blending with the halogenated hydantoin. However, iftwo or more waxes are mixed together, care should be taken because of the expected effect on the melting point (or melting point range) of the mixture, as compared to those of the waxes being mixed. Preferably, prior to compaction, the paraffin wax begins to melt at a temperature of at least about 50°C.

Generally, durable compacted articles can be formed from paraffin waxes from submicron-sized powders up to granules as large as about 3 U.S. standard mesh size. Preferably, the paraffin wax will have, prior to compaction, particles with a minimum particle size of at least about 10 microns. Similarly, preferred paraffin waxes typically have, prior to compaction, a maximum particle size of no greater than about 0.5 millimeter.

Among particularly preferred paraffin waxes are those which, prior to compaction, (a) begin to melt at a temperature of at least about 50°C, or (b) have a minimum particle size of at least about 10 microns, or (c) a maximum particle size of no greater than about 0.5 millimeter, or (d) have a combination of any two or all three of (a), (b), and (c).

Various methods can be used in forming the dry blends of this invention. Among preferred methods are use of ribbon blenders or tumble blenders for mixing the halogenated hydantoin and the paraffin wax. Equipment of this type is readily available in the marketplace from a number of reputable suppliers. As noted above, the amount of the paraffin wax in the dry blends of this invention will fall within the range of 0.5 to 10 wt%, and preferably in the range of one to 5 wt%, based on the total weight of the halogenated hydantoin and the paraffin wax. It will be understood and appreciated that departures from these ranges are permissible without departing from the scope of this invention, whenever such departures are deemed necessary or appropriate.

In forming the dry blends and compacted products of this invention, additional components can be included in order to partake of their desirable functions and characteristics. Such additional components, often termed excipients, include lubricants, disintegrants, and mold release agents. Other optional ingredients which may be used in the formulation of products include fragrances, stabilizers, adjuvants, corrosion inhibitors, dyes, surfactants, synergists, effervescents, diluents, builders, chelating agents, or buffers. Such ancillary materials should of course be compatible with the halogenated hydantoin(s) and not interfere in any material way with its performance characteristics.

Another feature of this invention is that powdery or finely-divided halogenated hydantoins, when blended with a suitable paraffin wax, can be converted directly into pressure compacted forms, such as caplets or tablets.

Preferred halogenated hydantoins in the practice of this invention are the 1,3-dihalo-5,5-dialkylhydantoins, especially 1,3-dibromo-5,5-dimethyl-hydantoin, 1,3-dichloro-5,5-dimethylhydantoin, 1-bromo-3-chloro-5,5-dimethylhydantoin, and 1-chloro-3-bromo-5,5-dimethylhydantoin, or mixtures of two or more of them. These are biocidal agents for use in water treatment. These compounds are, in general, sparingly soluble in water. Thus typically for water treatment applications 1,3-dichloro-5,5-dimethylhydantoin is supplied in the form of a puck for insertion into a toilet tank. These pucks apparently are formed by use of one or more binders, such as 1,3-dichloro-5-ethyl-5-methylhydantoin and/or ethylenebis(stearamide). N,N'-bromochloro-5,5-dimethylhydantoin is supplied in solid forms such as granules, tablets, or briquettes for delivery into the water being treated by means of water flow through an erosion feeder, or in the form of pucks for insertion into a toilet tank. Here again, these solid forms apparently are produced by use of one or more binders such as 1,3-dichloro-5-ethyl-5-methylhydantoin and/or ethylenebis(stearamide).

In converting the 1,3-dihalo-5,5-dimethylhydantoin/binder blends of this invention into granules, conventional processing equipment can be used under the usual operating conditions. Typically, the 1,3-dihalo-5,5-dimethylhydantoin/binder blend is compressed into sheet form by means of a roll compactor. This sheet in turn is broken up into small granules by a mechanical device, such as a Chilsonator^{®} breaker (The Fitzpatrick Company, Elmhurst, Illinois). The granules are then classified by screening into the desired size range. Undersized granules are typically recycled to the roll compactor, whereas oversized granules are recycled to the breaker device.

Average particle size and particle size distribution of the powdery or finely-divided substrate halogenated hydantoin can vary widely. The only true limitation is that the hydantoin(s) being compacted with the paraffin wax are not so large or of such character that despite the application of the compression pressure and the presence of the paraffin wax, such hydantoin(s) are incapable of being compacted and bound together into a durable form or shape. Typically this invention can be used successfully to form durable compacted articles from submicron-sized powders up to granules as large as about 3 U.S. standard mesh size. However, preferably the substrate hydantoin(s), such as for example one or more 1,3-dihalo-5,5-dialkylhydantoins, and especially 1,3-dibromo-5,5-dimethylhydantoin, being shaped or formed pursuant to this invention will have particles with a particle size in the range of from 20 microns up to 3 U.S. Standard mesh size. Typically the average particle size of such 1,3-dihalo-5,5-dialkylhydantoins will be in the range of 20 to 600 microns. Preferred for use with a binder of this invention is 1,3-dibromo-5,5-dimethylhydantoin particulate solids having an average particle size in the range of 175 to 400 microns. Nevertheless departures from these sizes are permissible whenever deemed desirable or appropriate, and thus are within the scope of this invention.

The formation of tablets and other compressed shapes such as briquettes from the blends of this invention can utilize conventionally known processing equipment and, for the most part, known procedures. However, in conducting compaction of the blends of this invention, it is important that the compaction pressure be sufficient to induce plastic deformation and interparticulate binding of the particles. At the same time, the compaction pressure should not be so great as to produce a compacted product which delaminates. Typically, suitable compaction pressures in the practice of this invention will fall within the range of 1000 to 30,000 psi, and preferably in the range of 5000 to 25,000 psi. Such compaction can be conducted using, for example, a rotary tableting press operated at conventional rotational speeds. Another method for accomplishing the compaction is by means of pressure extrusion through a die orifice, while concurrently shearing the extrudate to produce compacted shapes of the desired size. In such operations, the compaction pressures within the die should be sufficient to induce plastic deformation and interparticulate binding of the particles, but insufficient to produce a compacted product which, when extruded, undergoes an elastic recovery of a magnitude that causes delamination of the compacted extrudate.

When carrying out compaction of a blend of this invention, it is desirable, but not essential, to apply a pressure agglomeration lubricant to the compaction surfaces of the tooling so as to reduce the coefficient of friction between the material being compacted and the tooling. When using such lubricant, it is possible to utilize any of a variety of lubricants conventionally used for this purpose. However, a feature of this invention is that it is highly advantageous to employ, as such lubricant, a paraffin wax of the type described herein. Not only is the wax a highly effective lubricant, but in addition, the resultant compacted product is free from contamination by an additional component, namely a lubricant different from the paraffin wax.

In operations conducted on a small scale using manually filled dies, 1,3-dibromo-5,5-dimethylhydantoin/binder blends of this invention have been successfully compacted directly into tablets. The tablets when released from the dies were intact and exhibited no visual surface imperfections.

As also described above, this invention provides products in which one or more of the 1,3-dihalo-5,5-dimethylhydantoins blends of this invention are converted into granules, caplets, tablets, briquettes, pucks, or any other large size product, however produced. Typical operations of this type have been described above.

By the term "shape-retentive compacted composition" it is meant that the composition retains its shape after being compacted. The compacted composition may take a variety of shapes, often a geometrical shape. For example, the compacted composition can be a cylinder, rectangular solid, pyramid, or other polyhedral shape. These shape-retentive compacted compositions may be of any size, so long as they fit into typical compression equipment. Of course, the compacted composition should not be so large that, once formed, it will collapse under its own weight.

While there are no hard and fast rules governing differentiation with respect to size among granules, caplets, tablets, briquettes, and pucks, typically granules are regarded as being particles ranging in size from 80 to 3 U.S. standard mesh size. Caplets generally are in the range of 0.5 to 1 inch in length and with a cross-sectional width in the range of 0.25 to 0.5 inch. Tablets typically fall in the range of from 0.5 to 1.0 inch in diameter and 0.5 to 1.0 inch in thickness. Briquettes will normally range in size from 0.5 to 4.0 inches in length, from 0.5 to 4.0 inches in width, and from 0.5 to 2.5 inches in height. Pucks are normally disc-shaped objects having a diameter up to about 3.0 inches and a thickness in the range of 0.5 to 1.0 inch. It will be understood and appreciated however, that these dimensions are not intended to unduly limit the scope of this invention.

The following Examples are presented to illustrate the practice of, and advantages made possible by, this invention. These Examples are not intended to limit, and should not be construed as limiting, the scope of this invention to the particular operations or conditions described therein. In all operations described in the Examples in which tablets were produced, the interior surfaces of the die were lightly dusted with a hydrocarbon paraffin wax as a lubricant prior to filling the die with the powder to be compacted. In Table 1, the abbreviation DBDMH is used to represent 1,3-dibromo-5,5-dimethylhydantoin.

### Example 1

Blocks of hydrocarbon paraffin wax (melting point 56-61°C, Aldrich Company, Milwaukee, Wisconsin) were cut into small pieces with a knife and the wax was then crushed using a mortar and pestle. The mortar was then shaken to size-segregate the pulverized paraffin wax; the larger pieces of wax remained on top. These larger pieces of wax were removed with a spatula, and the remaining, smaller pieces of paraffin wax were used.

A portion of the smaller wax pieces (1.5 g) was weighed into a crystallizing dish, followed by 1,3-dibromo-5,5-dimethylhydantoin (48.5 g). A broad-bladed spatula was used to blend the mixture rather like a cook might blend butter into flour. After 10 minutes of hand mixing in this fashion, the mixture was subjected to a compaction test. The sample was weighed, and then introduced into a 0.71 inch diameter die made from Hastelloy^{®} C alloy and compacted using a screw-driven Sintech^{®} press (MTS Systems Corporation, Edenprairie, Minnesota) equipped with a punch also made of Hastelloy^{®} C alloy, to a pre-set pressure. Prior to filling the die, its interior surfaces were lightly dusted with a micronized polypropylene wax (MICROPRO 400 Micro Powders Inc., Tarrytown, New York) to serve as a lubricant. There was no dwell time upon attaining the compaction pressure (i. e., the pressure was released immediately). Upon extraction of the tablet from the die, a visual observation of the tablet was made.

### Example 2

Another portion of the crushed hydrocarbon paraffin wax from Example 1 (1.5 g) was weighed into a crystallizing dish, followed by 1,3-dibromo-5,5-dimethylhydantoin (48.5 g). This mixture was blended as described in Example 1, and subjected to a compaction test as described in Example 1.

### Example 3

Still another portion of the crushed hydrocarbon paraffin wax from Example 1 (1.5 g) was weighed into a crystallizing dish, followed by 1,3-dibromo-5,5-dimethylhydantoin (48.5 g). This mixture was blended as described in Example 1, and subjected to a compaction test as described in Example 1.

In all three Examples, the tablets were observed to have inhomogeneitites, which were attributed to the wide size range of the paraffin wax particles used.

For comparison, the compacted mixtures of Examples 1-3 were compared to compacted unblended virgin commercially produced 1,3-dibromo-5,5-dimethylhydantoin with an average particle size of about 64.5µ, and a commercial toilet bowl product (abbreviated in Table 1 as CTB product), which is known to be a mixture of other halogenated hydantoin compounds. This toilet bowl puck was purchased from a supermarket, ground to a powder with a mortar and pestle, and recompacted as above described.

Table 1 lists the experimental conditions and the observations.

**TABLE 1**

| Blend | Amount of blend added to die | Pressure | Observations |
|---|---|---|---|
| DBDMH/3 wt% paraffin wax | 5 g | 5000 psi | intact tablet, smooth shiny surfaces |
| DBDMH/3 wt% paraffin wax | 5 g | 5000 psi | intact tablet, smooth shiny surfaces |
| DBDMH/3 wt% paraffin wax | 5 g | 5000 psi | intact tablet, smooth shiny surfaces |
| DBDMH-without wax binder | 2.5g | 5000 psi | tablet shattered and laminated upon removal from die |
| CTB product | 2.5g | 5000 psi | intact tablet |

Compounds referred to by chemical name or formula anywhere in this document, whether referred to in the singular or plural, are identified as they exist prior to coming into contact with another substance referred to by chemical name or chemical type (*e.g*., another component, or a solvent). It matters not what preliminary chemical changes, if any, take place in the resulting mixture or solution, as such changes are the natural result of bringing the specified substances together under the conditions specified in this disclosure. Also, even though the claims may refer to substances in the present tense (*e.g*., "comprises" or "is"), the reference is to the substance as it exists at the time just before it is first contacted, blended or mixed with one or more other substances in accordance with the present disclosure.

## Claims

1. A shape-retentive compacted composition, which comprises a pressure compacted blend of a powdery or finely-divided halogenated hydantoin and a binder quantity of a paraffin wax, wherein said paraffin wax is a hydrocarbon paraffin wax and/or a chlorinated paraffin wax, said wax being compatible with said halogenated hydantoin.

2. A composition of Claim 1 wherein said wax is a hydrocarbon paraffin wax.

3. A composition of Claim 1 wherein said wax is a chlorinated paraffin wax.

4. A composition of Claim 1 wherein said wax is a mixture of hydrocarbon paraffin wax and chlorinated paraffin wax.

5. A composition of Claim 1 wherein said wax has, prior to compaction, a minimum particle size of at least about 10 microns.

6. A composition of Claim 1 wherein said wax has, prior to compaction, a maximum particle size of no greater than about 0.5 millimeter.

7. A composition of Claim 1 wherein, prior to compaction, said wax begins to melt at a temperature of at least about 50°C.

8. A composition of Claim 1 wherein said wax has, prior to compaction, a minimum particle size of at least about 10 microns, and a maximum particle size of no greater than about 0.5 millimeter.

9. A composition of Claim 8 wherein, prior to compaction, said wax begins to melt at a temperature of at least about 50°C.

10. A composition of Claim 1 wherein said wax is a hydrocarbon paraffin wax that, prior to compaction, begins to melt at a temperature of at least about 50°C.

11. A composition of Claim 1 wherein said wax is a hydrocarbon paraffin wax that has, prior to compaction, a minimum particle size of at least about 10 microns.

12. A composition of Claim 1 wherein said wax is a hydrocarbon paraffin wax that has, prior to compaction, a maximum particle size of no greater than about 0.5 millimeter.

13. A composition of Claim 1 wherein said wax is a hydrocarbon paraffin wax that, prior to compaction, begins to melt at a temperature of at least about 50°C, has a minimum particle size of at least about 10 microns, and has a maximum particle size of no greater than about 0.5 millimeter.

14. A composition of Claim 1 wherein said wax is a chlorinated paraffin wax that, prior to compaction, begins to melt at a temperature of at least about 50°C.

15. A composition of Claim 1 wherein said wax is a chlorinated paraffin wax that, prior to compaction, has a minimum particle size of at least about 10 microns.

16. A composition of Claim 1 wherein said wax is a chlorinated paraffin wax that, prior to compaction, has a maximum particle size of no greater than about 0.5 millimeter.

17. A composition of Claim 1 wherein said wax is a chlorinated paraffin wax that, prior to compaction, begins to melt at a temperature of at least about 50°C, has a minimum particle size of at least about 10 microns, and has a maximum particle size of no greater than about 0.5 millimeter.

18. A composition of Claim 1 wherein said wax is a mixture of hydrocarbon paraffin wax and chlorinated paraffin wax, and said mixture, prior to compaction, begins to melt at a temperature of at least about 50°C.

19. A composition of Claim 18 wherein said mixture has, prior to compaction, a minimum particle size of at least about 10 microns.

20. A composition of Claim 18 wherein said mixture has, prior to compaction, a maximum particle size of no greater than about 0.5 millimeter.

21. A composition of Claim 18 wherein said mixture, prior to compaction, has a minimum particle size of at least about 10 microns, and has a maximum particle size of no greater than about 0.5 millimeter.

22. A composition of any of Claims 1-8 wherein said binder quantity is in the range of 0.5 to 10 wt%, based on the total weight of said halogenated hydantoin and said wax.

23. A composition of any of Claims 1-8 wherein said binder quantity is in the range of one to 5 wt%, based on the total weight of said halogenated hydantoin and said wax.

24. A composition of Claim 1 wherein said halogenated hydantoin is a mono-N-halo-5,5-dialkylhydantoin in which the halogen atom is a chlorine or bromine atom.

25. A composition of Claim 1 wherein said halogenated hydantoin is an N,N'-dihalo-5,5-dialkylhydantoin in which each halogen atom is, independently, a chlorine or bromine atom.

26. A composition of Claim 25 wherein each alkyl group of said N,N'-dihalo-5,5-dialkylhydantoin contains, independently, in the range of one to 6 carbon atoms.

27. A composition of Claim 26 wherein said N,N'-dihalo-5,5-dialkylhydantoin is a 1,3-dichloro-5,5-dialkylhydantoin.

28. A composition of Claim 26 wherein said N,N'-dihalo-5,5-dialkylhydantoin is an N,N'-bromochloro-5,5-dialkylhydantoin.

29. A composition of Claim 26 wherein said N,N'-dihalo-5,5-dialkylhydantoin is a 1,3-dibromo-5,5-dialkylhydantoin.

30. A composition of Claim 26 wherein said N,N'-dihalo-5,5-dialkylhydantoin is 1,3-dichloro-5,5-dimethylhydantoin.

31. A composition of Claim 26 wherein said N,N'-dichloro-5,5-dialkylhydantoin is a mixture of 1,3-dichloro-5,5-dimethylhydantoin and 1,3-dichloro-5-ethyl-5-methyl-hydantoin.

32. A composition of Claim 26 wherein said N,N'-dihalo-5,5-dialkylhydantoin is an N,N'-bromochloro-5,5-dimethylhydantoin.

33. A composition of Claim 26 wherein said N,N'-dihalo-5,5-dialkylhydantoin is a mixture of N,N'-bromochloro-5,5-dimethylhydantoin and 1,3-dichloro-5-ethyl-5-methylhydantoin.

34. A composition of Claim 26 wherein said N,N'-dihalo-5,5-dialkylhydantoin is 1,3-dibromo-5,5-dimethylhydantoin.

35. A composition of Claim 26 wherein said N,N'-dihalo-5,5-dialkylhydantoin is a 1,3-dichloro-5,5-dialkylhydantoin, an N,N'-bromochloro-5,5-dialkylhydantoin, and/or a 1,3-dibromo-5,5-dialkylhydantoin, and wherein said wax is chlorinated paraffin wax.

36. A composition of Claim 35 wherein said wax has, prior to compaction, a minimum particle size of at least about 10 microns.

37. A composition of Claim 3 5 wherein said wax has, prior to compaction, a maximum particle size of no greater than about 0.5 millimeter.

38. A composition of Claim 35 wherein said wax, prior to compaction, begins to melt at a temperature of at least about 50°C.

39. A composition of Claim 35 wherein said wax has, prior to compaction, a minimum particle size of at least about 10 microns, a maximum particle size of no greater than about 0.5 millimeter, and wherein said wax, prior to compaction, begins to melt at a temperature of at least about 50°C.

40. A composition of Claim 26 wherein said N,N'-dihalo-5,5-dialkylhydantoin is:
a) 1,3-dichloro-5,5-dimethylhydantoin;
b) a mixture of 1,3-dichloro-5,5-dimethylhydantoin and 1,3-dichloro-5-ethyl-5-methylhydantoin;
c) an N,N'-bromochloro-5,5-dimethylhydantoin;
d) a mixture of N,N'-bromochloro-5,5-dimethylhydantoin and 1,3-dichloro-5-ethyl-5-methylhydantoin; or
e) 1,3-dibromo-5,5-dimethylhydantoin; and
wherein said wax is a hydrocarbon paraffin wax.

41. A composition of Claim 40 wherein said wax, prior to compaction, has a minimum particle size of at least about 10 microns.

42. A composition of Claim 40 wherein said wax, prior to compaction, has a maximum particle size of no greater than about 0.5 millimeter.

43. A composition of Claim 40 wherein said wax, prior to compaction, begins to melt at a temperature of at least about 50°C.

44. A composition of Claim 40 wherein said wax, prior to compaction, has a minimum particle size of at least about 10 microns, a maximum particle size of no greater than about 0.5 millimeter, and wherein said wax, prior to compaction, begins to melt at a temperature of at least about 50°C.

45. A composition of Claim 1 wherein said halogenated hydantoin has an average particle size ofless than about 300 microns, and is 1,3-dichloro-5,5-dimethylhydantoin, 1,3-dichloro-5-ethyl-5-methylhydantoin, an N,N'-bromochloro-5,5-dimethylhydantoin, or 1,3-dibromo-5,5-dimethylhydantoin, or a mixture of any two or more of said hydantoins.

46. A composition of Claim 45 wherein said wax is a hydrocarbon paraffin wax.

47. A composition of Claim 1 wherein said halogenated hydantoin, prior to compaction, has an average particle size of less than about 200 microns, and is 1,3-dichloro-5,5-dimethylhydantoin, 1,3-dichloro-5-ethyl-5-methylhydantoin, an N,N'-bromochloro-5,5-dimethylhydantoin, or 1,3-dibromo-5,5-dimethylhydantoin, or a mixture of any two or more of said hydantoins.

48. A composition of Claim 47 wherein said wax is a hydrocarbon paraffin wax.

49. A composition of Claim 1 wherein said halogenated hydantoin has an average particle size of at least about 300 microns prior to compaction, and is 1,3-dihalo-5,5-dimethylhydantoin in which each halogen atom is, independently, a chlorine atom or a bromine atom, and wherein said wax, prior to compaction, has a minimum particle size of at least about 10 microns, and a maximum particle size of no greater than about 0.5 millimeter.

50. A composition of Claim 49 wherein said wax is a hydrocarbon paraffin wax.

51. A method of producing a shape-retentive compacted composition as in claim 1, which method comprises pressure compacting a blend of a powdery or finely-divided halogenated hydantoin and a binder quantity of a paraffin wax, wherein said paraffin wax is a hydrocarbon paraffin wax and/or a chlorinated paraffin wax, said wax being compatible with said halogenated hydantoin.

52. A method of Claim 51 wherein said wax has, prior to compaction, a minimum particle size of at least about 10 microns.

53. A method of Claim 51 wherein said wax has, prior to compaction, a maximum particle size of no greater than about 0.5 millimeter.

54. A method of Claim 51 wherein said wax, prior to compaction, begins to melt at a temperature of at least about 50°C.

55. A method of Claim 51 wherein said wax, prior to compaction, has a minimum particle size of at least about 10 microns, and a maximum particle size of no greater than about 0.5 millimeter.

56. A method of any of Claims 51-55 wherein said blend is formed by tumble blending said powdery or finely-divided halogenated hydantoin and said paraffin wax to produce a substantially uniform blend.

57. A method of any of Claims 51-55 wherein said blend is formed by ribbon blending said powdery or finely-divided halogenated hydantoin and said paraffin wax to produce a substantially uniform blend.

58. A method of Claim 51 wherein said pressure compacting is conducted at a pressure in the range of 1000 to 30,000 psi.

59. Use of a dry blend in the production of a pressure compacted, shape-retentive composition as defined in claim 1.

60. Use according to Claim 59 wherein the amount of said wax is effective to form a shape-retentive article when said blend is subjected to pressure compaction.

61. Use according to Claim 60 wherein said blend further comprises at least one excipient or carrier.

62. Use according to any of Claims 59-61 wherein the halogenated hydantoin is 1,3-dihalo-5,5-dialkylhydantoin.

63. Use according to Claim 59 wherein said halogenated hydantoin is 1,3-dibromo-5,5-dimethylhydantoin, wherein the wax is a hydrocarbon paraffin wax, and wherein the amount of the wax is in the range of 0.5 to 10wt.%, preferably one to 5 wt% based on the total weight of the 1,3-dibromo-5,5-dimethylhydantoin and the wax.

64. Use according to Claim 63 wherein said hydrocarbon paraffin wax, prior to compaction, begins to melt at a temperature of at least about 50°C.

65. Use according to Claim 63 wherein said hydrocarbon paraffin wax has, prior to compaction, a minimum particle size of at least about 10 microns.

66. Use according to Claim 63 wherein said hydrocarbon paraffin wax has, prior to compaction, a maximum particle size of no greater than about 0.5 millimeter.

67. Use according to Claim 63 wherein said hydrocarbon paraffin wax, prior to compaction, begins to melt at a temperature of at least about 50°C, has a minimum particle size of at least about 10 microns, and has a maximum particle size of no greater than about 0.5 millimeter.

68. A compacted form or shape formed by pressure compacting a blend of any of Claims 63-67.

69. A compacted composition as in claim 1 which is a granule, caplet, tablet briquette or puck formed by pressure compacting a dry mixture consisting essentially of (i) 1,3-dibromo-5,5-dimethylhydantoin particulate solids, and (ii) a hydrocarbon paraffin wax in an amount in the range of 0.5 to 10 wt.% based on the total weight of the 1,3-dibromo-5,5-dimethylhydantoin and the wax.

70. A compacted compostion of claim 69 wherein said compacted composition is a tablet, wherein said 1,3-dibromo-5,5-dimethylhydantoin particulate solids have an average particle size in the range of 125 to 300 microns, and wherein said amount is in the range of 1 to 5 wt.% based on the total weight of 1,3-dibromo-5,5-dimethylhydantoin and the wax.

71. A composition of claims 69 or 70 wherein said hydrocarbon paraffin wax, prior to compaction, begins to melt at a temperature of at least about 50 °C, has a minimum particle size of at least about 10 microns, and has a maximum particles size of not greater than about 0.5 millimeter.

## Patentansprüche

1. Formstabile, verdichtete Zusammensetzung, die eine druckverdichtete Mischung aus pulverigem oder feinteiligem, halogeniertem Hydantoin und einer Bindemittelmenge Paraffinwachs umfasst, wobei das Paraffinwachs ein Kohlenwasserstoffparaffinwachs und/oder ein chloriertes Paraffinwachs ist, wobei das Wachs mit dem halogenierten Hydantoin verträglich ist.

2. Zusammensetzung nach Anspruch 1, bei der das Wachs ein Kohlenwasserstoffparaffinwachs ist.

3. Zusammensetzung nach Anspruch 1, bei der das Wachs ein chloriertes Paraffinwachs ist.

4. Zusammensetzung nach Anspruch 1, bei der das Wachs eine Mischung von Kohlenwasserstoffparaffinwachs und chloriertem Paraffinwachs ist.

5. Zusammensetzung nach Anspruch 1, bei der das Wachs vor der Verdichtung eine minimale Teilchengröße von mindestens etwa 10 µm aufweist.

6. Zusammensetzung nach Anspruch 1, bei der das Wachs vor der Verdichtung eine maximale Teilchengröße von nicht größer als etwa 0,5 mm aufweist.

7. Zusammensetzung nach Anspruch 1, bei der vor der Verdichtung das Wachs bei einer Temperatur von mindestens etwa 50 °C zu schmelzen beginnt.

8. Zusammensetzung nach Anspruch 1, bei der das Wachs vor der Verdichtung eine minimale Teilchengröße von mindestens etwa 10 µm und eine maximale Teilchengröße von nicht größer als etwa 0,5 mm aufweist.

9. Zusammensetzung nach Anspruch 8, bei der das Wachs vor der Verdichtung bei einer Temperatur von mindestens etwa 50 °C zu schmelzen beginnt.

10. Zusammensetzung nach Anspruch 1, bei der das Wachs ein Kohlenwasserstoffparaffinwachs ist, das vor der Verdichtung bei einer Temperatur von mindestens etwa 50 °C zu schmelzen beginnt.

11. Zusammensetzung nach Anspruch 1, bei der das Wachs ein Kohlenwasserstoffparaffinwachs ist, das vor der Verdichtung eine minimale Teilchengröße von mindestens etwa 10 µm aufweist.

12. Zusammensetzung nach Anspruch 1, bei der das Wachs ein Kohlenwasserstoffparaffinwachs ist, das vor der Verdichtung eine maximale Teilchengröße von nicht größer als etwa 0,5 mm aufweist.

13. Zusammensetzung nach Anspruch 1, bei der das Wachs ein Kohlenwasserstoffparaffinwachs ist, das vor der Verdichtung bei einer Temperatur von mindestens etwa 50 °C zu schmelzen beginnt, eine minimale Teilchengröße von mindestens etwa 10 µm aufweist und eine maximale Teilchengröße von nicht größer als etwa 0,5 mm aufweist.

14. Zusammensetzung nach Anspruch 1, bei der das Wachs ein chloriertes Paraffinwachs ist, das vor der Verdichtung bei einer Temperatur von mindestens etwa 50 °C zu schmelzen beginnt.

15. Zusammensetzung nach Anspruch 1, bei der das Wachs ein chloriertes Paraffinwachs ist, das vor der Verdichtung eine minimale Teilchengröße von mindestens etwa 10 µm aufweist.

16. Zusammensetzung nach Anspruch 1, bei der das Wachs ein chloriertes Paraffinwachs ist, das vor der Verdichtung eine maximale Teilchengröße von nicht größer als etwa 0,5 mm aufweist.

17. Zusammensetzung nach Anspruch 1, bei der das Wachs ein chloriertes Paraffinwachs ist, das vor der Verdichtung bei einer Temperatur von mindestens etwa 50 °C zu schmelzen beginnt, eine minimale Teilchengröße von mindestens etwa 10 µm aufweist und eine maximale Teilchengröße von nicht größer als etwa 0,5 mm aufweist.

18. Zusammensetzung nach Anspruch 1, bei der das Wachs eine Mischung von Kohlenwasserstoffparaffinwachs und chloriertem Paraffinwachs ist und die Mischung vor der Verdichtung bei einer Temperatur von mindestens etwa 50 °C zu schmelzen beginnt.

19. Zusammensetzung nach Anspruch 18, bei der die Mischung vor der Verdichtung eine minimale Teilchengröße von mindestens etwa 10 µm aufweist.

20. Zusammensetzung nach Anspruch 18, bei der die Mischung vor der Verdichtung eine maximale Teilchengröße von nicht mehr als etwa 0,5 mm aufweist.

21. Zusammensetzung nach Anspruch 18, bei der die Mischung vor der Verdichtung eine minimale Teilchengröße von mindestens etwa 10 µm aufweist und eine maximale Teilchengröße von nicht größer als etwa 0,5 mm aufweist.

22. Zusammensetzung nach einem der Ansprüche 1 bis 8, bei der die Bindemittelmenge, bezogen auf das Gesamtgewicht des halogenierten Hydantoins und des Wachses, im Bereich von 0,5 bis 10 Gew.-% liegt.

23. Zusammensetzung nach einem der Ansprüche 1 bis 8, bei der die Bindemittelmenge, bezogen auf das Gesamtgewicht des halogenierten Hydantoins und des Wachses, im Bereich von 1 bis 5 Gew.-% liegt.

24. Zusammensetzung nach Anspruch 1, bei der das halogenierte Hydantoin ein Mono-N-halogen-5,5-dialkylhydantoin ist, bei dem das Halogenatom ein Chlor- oder Bromatom ist.

25. Zusammensetzung nach Anspruch 1, bei der das halogenierte Hydantoin ein N,N'-Dihalogen-5,5-dialkylhydantoin ist, bei dem jedes Halogenatom jeweils unabhängig ein Chlor- oder Bromatom ist.

26. Zusammensetzung nach Anspruch 25, bei der jede Alkylgruppe des N,N'-Dihalogen-5,5-dialkylhydantoins jeweils unabhängig 1 bis 6 Kohlenstoffatome enthält.

27. Zusammensetzung nach Anspruch 26, bei der das N,N'-Dihalogen-5,5-dialkylhydantoin ein 1,3-Dichlor-5,5-dialkylhydantoin ist.

28. Zusammensetzung nach Anspruch 26, bei der das N,N'-Dihalogen-5,5-dialkylhydantoin ein N,N'-Bromchlor-5,5-dialkylhydantoin ist.

29. Zusammensetzung nach Anspruch 26, bei der das N,N'-Dihalogen-5,5-dialkylhydantoin ein 1,3-Dibrom-5,5-dialkylhydantoin ist.

30. Zusammensetzung nach Anspruch 26, bei der das N,N'-Dihalogen-5,5-dialkylhydantoin ein 1,3-Dichlor-5,5-dimethylhydantoin ist.

31. Zusammensetzung nach Anspruch 26, bei der das N,N'-Dichlor-5,5-dialkylhydantoin eine Mischung von 1,3-Dichlor-5,5-dimethylhydantoin und 1,3-Dichlor-5-ethyl-5-methylhydantoin ist.

32. Zusammensetzung nach Anspruch 26, bei der das N,N'-Dihalogen-5,5-dialkylhydantoin ein N,N'-Bromchlor-5,5-dimethylhydantoin ist.

33. Zusammensetzung nach Anspruch 26, bei der das N,N'-Dihalogen-5,5-dialkylhydantoin eine Mischung von N,N'-Bromchlor-5,5-dimethylhydantoin und 1,3-Dichlor-5-ethyl-5-methylhydantoin ist.

34. Zusammensetzung nach Anspruch 26, bei der das N,N'-Dihalogen-5,5-dialkylhydantoin 1,3-Dibrom-5,5-dimethylhydantoin ist.

35. Zusammensetzung nach Anspruch 26, bei der das N,N'-Dihalogen-5,5-dialkylhydantoin ein 1,3-Dichlor-5,5-dialkylhydantoin, ein N,N'-Bromchlor-5,5-dialkylhydantoin und/oder ein 1,3-Dibrom-5,5-dialkylhydantoin ist und das Wachs chloriertes Paraffinwachs ist.

36. Zusammensetzung nach Anspruch 35, bei der das Wachs vor der Verdichtung eine minimale Teilchengröße von mindestens etwa 10 µm aufweist.

37. Zusammensetzung nach Anspruch 35, bei der das Wachs vor der Verdichtung eine minimale Teilchengröße von nicht größer als etwa 0,5 mm aufweist.

38. Zusammensetzung nach Anspruch 35, bei der das Wachs vor der Verdichtung bei einer Temperatur von mindestens etwa 50 °C zu schmelzen beginnt.

39. Zusammensetzung nach Anspruch 35, bei der das Wachs vor der Verdichtung eine minimale Teilchengröße von mindestens etwa 10 µm aufweist, eine maximale Teilchengröße von nicht größer als etwa 0,5 mm aufweist und das Wachs vor der Verdichtung bei einer Temperatur von mindestens etwa 50 °C zu schmelzen beginnt.

40. Zusammensetzung nach Anspruch 26, bei der das N,N'-Dihalogen-5,5-dialkylhydantoin:
a) 1,3-Dichlor-5,5-dimethylhydantoin,
b) eine Mischung von 1,3-Dichlor-5,5-dimethylhydantoin und 1,3-Dichlor-5-ethyl-5-methylhydantoin,
c) N,N'-Bromchlor-5,5-dimethylhydantoin,
d) eine Mischung von N,N'-Bromchlor-5,5-dimethylhydantoin und 1,3-Dichlor-5-ethyl-5-methylhydantoin oder
e) 1,3-Dibrom-5,5-dimethylhydantoin ist, und
bei der das Wachs ein Kohlenwasserstoffparaffinwachs ist.

41. Zusammensetzung nach Anspruch 40, bei der das Wachs vor der Verdichtung eine minimale Teilchengröße von mindestens etwa 10 µm aufweist.

42. Zusammensetzung nach Anspruch 40, bei der das Wachs vor der Verdichtung eine maximale Teilchengröße von nicht größer als etwa 0,5 mm aufweist.

43. Zusammensetzung nach Anspruch 40, bei der das Wachs vor der Verdichtung bei einer Temperatur von mindestens etwa 50 °C zu schmelzen beginnt.

44. Zusammensetzung nach Anspruch 40, bei der das Wachs vor der Verdichtung eine minimale Teilchengröße von mindestens etwa 10 µm, eine maximale Teilchengröße von nicht größer als etwa 0,5 mm aufweist und das Wachs vor der Verdichtung bei einer Temperatur von mindestens etwa 50 °C zu schmelzen beginnt.

45. Zusammensetzung nach Anspruch 1, bei der das halogenierte Hydantoin eine durchschnittliche Teilchengröße von weniger als etwa 300 µm aufweist und 1,3-Dichlor-5,5-dimethylhydantoin, 1,3-Dichlor-5-ethyl-5-methylhydantoin, N,N'-Bromchlor-5,5-dimethylhydantoin oder 1,3-Dibrom-5,5-dimethylhydantoin oder eine Mischung von irgendwelchen zwei oder mehr dieser Hydantoine ist.

46. Zusammensetzung nach Anspruch 45, bei der das Wachs ein Kohlenwasserstoffparaffinwachs ist.

47. Zusammensetzung nach Anspruch 1, bei der das halogenierte Hydantoin vor der Verdichtung eine durchschnittliche Teilchengröße von weniger als etwa 200 µm aufweist und 1, 3-Dichlor-5,5-dimethylhydantoin, 1,3-Dichlor-5-ethyl-5-methylhydantoin, N,N'-Bromchlor-5,5-dimethylhydantoin oder 1,3-Dibrom-5,5-dimethylhydantoin oder eine Mischung von irgendwelchen zwei oder mehr dieser Hydantoine ist.

48. Zusammensetzung nach Anspruch 47, bei der das Wachs ein Kohlenwasserstoffparaffinwachs ist.

49. Zusammensetzung nach Anspruch 1, bei der das halogenierte Hydantoin vor der Verdichtung eine durchschnittliche Teilchengröße von mindestens etwa 300 µm aufweist und 1,3-Dihalogen-5,5-dimethylhydantoin ist, bei dem jedes Halogenatom jeweils unabhängig ein Chloratom oder ein Bromatom ist, und bei der das Wachs vor der Verdichtung eine minimale Teilchengröße von mindestens etwa 10 µm und eine maximale Teilchengröße von nicht größer als etwa 0,5 mm aufweist.

50. Zusammensetzung nach Anspruch 49, bei der das Wachs ein Kohlenwasserstoffparaffinwachs ist.

51. Verfahren zur Herstellung einer formstabilen, verdichteten Zusammensetzung gemäß Anspruch 1, bei dem eine Mischung aus pulvrigem oder feinteiligem, halogeniertem Hydantoin und einer Bindemittelmenge Paraffinwachs druckverdichtet wird, wobei das Paraffinwachs ein Kohlenwasserstoffparaffinwachs und/oder ein chloriertes Paraffinwachs ist, wobei das Wachs mit dem halogenierten Hydantoin verträglich ist.

52. Verfahren nach Anspruch 51, bei dem das Wachs vor der Verdichtung eine minimale Teilchengröße von mindestens etwa 10 µm aufweist.

53. Verfahren nach Anspruch 51, bei dem das Wachs vor der Verdichtung eine maximale Teilchengröße von nicht größer als etwa 0,5 mm aufweist.

54. Verfahren nach Anspruch 51, bei der das Wachs vor der Verdichtung bei einer Temperatur von mindestens etwa 50 °C zu schmelzen beginnt.

55. Verfahren nach Anspruch 51, bei dem das Wachs vor der Verdichtung eine minimale Teilchengröße von mindestens etwa 10 µm und eine maximale Teilchengröße von nicht größer als etwa 0,5 mm aufweist.

56. Verfahren nach einem der Ansprüche 51 bis 55, bei dem die Mischung durch Trommelmischen des pulvrigen oder feinteiligen, halogenierten Hydantoins und des Paraffinwachses gebildet wird, um eine im Wesentlichen gleichförmige Mischung herzustellen.

57. Verfahren nach einem der Ansprüche 51 bis 55, bei dem die Mischung durch Bandmischen des pulvrigen oder feinteiligen, halogenierten Hydantoins und des Paraffinwachses gebildet wird, um eine im Wesentlichen gleichförmige Mischung herzustellen.

58. Verfahren nach einem der Ansprüche 51, bei dem die Druckverdichtung bei einem Druck im Bereich von 1000 bis 30 000 psi durchgeführt wird.

59. Verwendung einer trockenen Mischung bei der Herstellung einer druckverdichteten, formbeständigen Zusammensetzung gemäß Anspruch 1.

60. Verwendung nach Anspruch 59, bei der die Menge des Wachses wirksam ist, um einen formbeständigen Gegenstand zu bilden, wenn die Mischung einer Druckverdichtung unterzogen wird.

61. Verwendung nach Anspruch 60, bei der die Mischung ferner mindestens ein Hilfsmittel oder einen Träger umfasst.

62. Verwendung nach einem der Ansprüche 59 bis 61, bei der das halogenierte Hydantoin 1,3-Dihalogen-5,5-dialkylhydantoin ist.

63. Verwendung nach Anspruch 59, bei der das halogenierte Hydantoin 1,3-Dibrom-5,5-dimethylhydantoin ist, das Wachs ein Kohlenwasserstoffparaffinwachs ist und die Menge des Wachses, bezogen auf das Gesamtgewicht des 1,3-Dibrom-5,5-dimethylhydantoins und des Wachses, im Bereich von 0,5 bis 10 Gew.-%, vorzugsweise 1 bis 5 Gew.-% liegt.

64. Verwendung nach Anspruch 63, bei der das Kohlenwasserstoffparaffinwachs vor der Verdichtung bei einer Temperatur von mindestens etwa 50 °C zu schmelzen beginnt.

65. Verwendung nach Anspruch 63, bei der das Kohlenwasserstoffparaffinwachs vor der Verdichtung eine minimale Teilchengröße von mindestens etwa 10 µm aufweist.

66. Verwendung nach Anspruch 63, bei der das Kohlenwasserstoffparaffinwachs vor der Verdichtung eine maximale Teilchengröße von größer als etwa 0,5 mm aufweist.

67. Verwendung nach Anspruch 63, bei der das Kohlenwasserstoffparaffinwachs vor der Verdichtung bei einer Temperatur von mindestens etwa 50 °C zu schmelzen beginnt, eine minimale Teilchengröße von mindestens etwa 10 µm aufweist und eine maximale Teilchengröße von nicht größer als etwa 0,5 mm aufweist.

68. Verdichtete Form oder Gestalt, die durch Druckverdichtung einer Mischung gemäß einem der Ansprüche 63 bis 67 hergestellt worden ist.

69. Verdichtete Zusammensetzung nach Anspruch 1, die ein Korn, eine Kapsel, eine Tablette, ein Brikett oder ein Puck ist, das/die/der durch Druckverdichtung der trockenen Mischung gebildet worden ist, die im Wesentlichen aus (i) teilchenförmigen 1,3-Dibrom-5,5-dimethylhydantoin Feststoffen und (ii) Kohlenwasserstoffparaffinwachs in einer Menge, bezogen auf das Gesamtgewicht des 1,3-Dibrom-5,5-dimethylhydantoins und des Wachses, im Bereich von 0,5 bis 10 Gew.-% besteht.

70. Verdichtete Zusammensetzung nach Anspruch 69, bei der die verdichtete Zusammensetzung ein Tablette ist, bei der die teilchenförmigen 1,3-Dibrom-5,5-dimethylhydantoin Feststoffe eine durchschnittliche Teilchengröße im Bereich von 125 bis 300 µm aufweisen und die Menge, bezogen auf das Gesamtgewicht des 1,3-Dibrom-5,5-dimethylhydantoins und des Wachses, im Bereich von 1 bis 5 Gew.-% liegt.

71. Verdichtete Zusammensetzung nach Anspruch 69 oder 70, bei der das Kohlenwasserstoffparaffinwachs vor der Verdichtung bei einer Temperatur von mindestens etwa 50 °C zu schmelzen beginnt, eine minimale Teilchengröße von mindestens etwa 10 µm aufweist und eine maximale Teilchengröße von nicht größer als etwa 0,5 mm aufweist.

## Revendications

1. Composition compacte à mémoire de forme qui comprend un mélange compacté par pression d'une hydantoïne halogénée poudreuse ou finement divisée et d'une quantité d'un liant de cire de paraffine, dans laquelle ladite cire de paraffine est une cire de paraffine hydrocarbonée et/ou une cire de paraffine chlorée, ladite cire étant compatible avec ladite hydantoïne halogénée.

2. Composition selon la revendication 1, dans laquelle ladite cire est une cire de paraffine hydrocarbonée.

3. Composition selon la revendication 1, dans laquelle ladite cire est une cire de paraffine chorée.

4. Composition selon la revendication 1, dans laquelle ladite cire est un mélange de cire de paraffine hydrocarbonée et de cire de paraffine chlorée.

5. Composition selon la revendication 1, dans laquelle ladite cire présente, avant compactage, une taille minimale de particules d'au moins environ 10 microns.

6. Composition selon la revendication 1, dans laquelle ladite cire présente, avant compactage, une taille maximale de particules qui n'est pas supérieure à environ 0,5 mm.

7. Composition selon la revendication 1, dans laquelle avant compactage, ladite cire commence à fondre à une température d'au moins environ 50°C.

8. Composition selon la revendication 1, dans laquelle ladite cire présente, avant compactage, une taille de particules minimale d'au moins environ 10 microns et une taille de particules maximale qui n'est pas supérieure à environ 0,5 mm.

9. Composition selon la revendication 8, dans laquelle avant compactage, ladite cire commence à fondre à une température d'au moins environ 50°C.

10. Composition selon la revendication 1, dans laquelle ladite cire est une cire de paraffine hydrocarbonée qui, avant compactage, commence à fondre à une température d'au moins environ 50°C.

11. Composition selon la revendication 1, dans laquelle ladite cire est une cire de paraffine hydrocarbonée qui présente, avant compactage, une taille de particules minimale d'au moins environ 10 microns.

12. Composition selon la revendication 1, dans laquelle ladite cire est une cire de paraffine hydrocarbonée qui présente, avant compactage, une taille de particules qui n'est pas supérieure à environ 0,5 mm.

13. Composition selon la revendication 1, dans laquelle ladite cire est une cire de paraffine hydrocarbonée, qui avant compactage, commence à fondre à une température d'au moins environ 50°C, présente une taille minimale de particules d'au moins environ 10 microns et présente une taille de particules maximale qui n'est pas supérieure à environ 0,5 mm.

14. Composition selon la revendication 1, dans laquelle ladite cire est une cire de paraffine chlorée qui, avant compactage, commence à fondre à une température d'au moins environ 50°C.

15. Composition selon la revendication 1, dans laquelle ladite cire est une cire de paraffine chlorée qui, avant compactage, présente une taille minimale de particules d'au moins environ 10 microns.

16. Composition selon la revendication 1, dans laquelle ladite cire est une cire de paraffine chlorée qui, avant compactage, présente une taille maximale de particules qui n'est pas supérieure à environ 0,5 mm.

17. Composition selon la revendication 1, dans laquelle ladite cire est une cire de paraffine chlorée qui, avant compactage, commence à fondre à une température d'au moins environ 50°C, présente une taille minimale de particules d'au moins environ 10 microns, et présente une taille maximale de particules qui n'est pas supérieure à environ 0,5 mm.

18. Composition selon la revendication 1, dans laquelle ladite cire est un mélange de cire de paraffine hydrocarbonée et de cire de paraffine chlorée, et ledit mélange, avant compactage, commence à fondre à une température d'au moins environ 50°C.

19. Composition selon la revendication 18, dans laquelle ledit mélange présente, avant compactage, une taille minimale de particules d'au moins environ 10 microns.

20. Composition selon la revendication 18, dans laquelle ledit mélange présente, avant compactage, une taille maximale de particules qui n'est pas supérieure à environ 0,5 mm.

21. Composition selon la revendication 18, dans laquelle ledit mélange présente, avant compactage, une taille minimale de particules d'au moins environ 10 microns et présente une taille maximale de particules qui n'est pas supérieure à environ 0,5 mm.

22. Composition selon l'une quelconque des revendications 1 à 8, dans laquelle la quantité de liant est dans l'intervalle de 0,5 à 10 % en poids, du poids total de ladite hydantoïne halogénée et de ladite cire.

23. Composition selon l'une quelconque des revendications 1 à 8, dans laquelle la quantité de liant est dans l'intervalle de 1 à 5 % en poids du poids total de ladite hydantoïne halogénée et de ladite cire.

24. Composition selon la revendication 1, dans laquelle ladite hydantoïne halogénée est la mono-N-halo-5,5-dialkylhydantoïne dans laquelle l'atome d'halogène est un atome de chlore ou de brome.

25. Composition selon la revendication 1, **caractérisée en que** ladite hydantoïne halogénée est une N,N'-dihalo-5,5-dialkylhydantoïne dans laquelle chaque atome d'halogène est, indépendamment, un atome de chlore ou de brome.

26. Composition selon la revendication 25, dans laquelle chaque groupe alkyle de ladite N,N'-dihalo-5,5-dialkylhydantoïne contient, indépendamment, dans la plage de 1 à 6 atomes de carbone.

27. Composition selon la revendication 26, dans laquelle ladite N,N'-dihalo-5,5-dialkylhydantoïne est une 1,3-dichloro-5,5-dialkylhydantoïne.

28. Composition selon la revendication 26, dans laquelle ladite N,N'-dihalo-5,5-dialkylhydantoïne est une N,N'-bromochloro-5,5-dialkylhydantoïne.

29. Composition selon la revendication 26, dans laquelle ladite N,N'-dihalo-5,5-dialkylhydantoïne est une 1,3-dibromo-5,5-dialkylhydantoïne.

30. Composition selon la revendication 26, dans laquelle ladite N,N'-dihalo-5,5-dialkylhydantoïne est la 1,3-dichloro-5,5-diméthylhydantoïne.

31. Composition selon la revendication 26, dans laquelle ladite N,N'-dichloro-5,5-dialkylhydantoïne est un mélange de 1,3-dichloro-5,5-diméthylhydantoïne et de 1,3-dichloro-5-éthyl-5-méthyl-hydantoïne.

32. Composition selon la revendication 26, dans laquelle ladite N,N'-dihalo-5,5-dialkylhydantoïne est une N,N'-bromochloro-5,5-diméthylhydantoïne.

33. Composition selon la revendication 26, dans laquelle ladite N,N'-dihalo-5,5-dialkylhydantoïne est un mélange de N,N'-bromochloro-5,5-diméthylhydantoïne et de 1,3-dichloro-5-éthyl-5-méthylhydantoïne.

34. Composition selon la revendication 26, dans laquelle ladite N,N'-dihalo-5,5-dialkylhydantoïne est la 1,3-dibromo-5,5-diméthylhydantoine.

35. Composition selon la revendication 26, dans laquelle ladite N,N'-dihalo-5,5-dialkylhydantoïne est une 1,3-dichloro-5,5-dialkylhydantoïne, une N,N'-bromochloro-5,5-dialkylhydantoïne, et/ou une 1,3-dibromo-5,5-dialkylhydantoïne, et dans laquelle ladite cire est une cire de paraffine chlorée.

36. Composition selon la revendication 35, dans laquelle ladite cire présente, avant compactage, une taille de particules minimale d'au moins environ 10 microns.

37. Composition selon la revendication 35, dans laquelle ladite cire présente, avant compactage, une taille maximale de particules qui n'est pas supérieure à environ 0,5 mm.

38. Composition selon la revendication 35, dans laquelle ladite cire, avant compactage, commence à fondre à une température d'au moins environ 50°C.

39. Composition selon la revendication 35, dans laquelle ladite cire présente, avant compactage, une taille minimale de particules d'au moins environ 10 microns, une taille maximale de particules qui n'est pas supérieure à environ 0,5 mm, et dans laquelle ladite cire, avant compactage, commence à fondre à une température d'au moins environ 50°C.

40. Composition selon la revendication 26, dans laquelle ladite N,N'-dihalo-5,5-dialkylhydantoïne est :
a) la 1,3-dichloro-5,5-diméthylhydantoïne ;
b) un mélange de 1,3-dichloro-5,5-diméthylhydantoïne et de 1,3-dichloro-5-éthyl-5-méthylhydantoïne ;
c) une N,N'-bromochloro-5,5-diméthylhydantoïne ;
d) un mélange de N,N'-bromochloro-5,5-diméthylhydantoïne et de 1,3-dichloro-5-éthyl-5-méthylhydantoïne ; ou
e) la 1,3-dibromo-5,5-diméthylhydantoïne ; et dans laquelle ladite cire est une cire de paraffine hydrocarbonée.

41. Composition selon la revendication 40, dans laquelle ladite cire, avant compactage, présente une taille de particules minimale d'au moins environ 10 microns.

42. Composition selon la revendication 40, dans laquelle ladite cire présente, avant compactage, une taille maximale de particules qui n'est pas supérieure à environ 0,5 mm.

43. Composition selon la revendication 40, dans laquelle ladite cire, avant compactage, commence à fondre à une température d'au moins environ 50°C.

44. Composition selon la revendication 40, dans laquelle ladite cire, avant compactage, présente une taille minimale de particules d'au moins environ 10 microns, une taille maximale de particules qui n'est pas supérieure à environ 0,5 mm, et dans laquelle ladite cire, avant compactage, commence à fondre à une température d'au moins environ 50°C.

45. Composition selon la revendication 1, dans laquelle ladite hydantoïne halogénée présente une taille moyenne de particules inférieure à environ 300 microns, et est la 1,3-dichloro-5,5-diméthylhydantoïne, 1,3-dichloro-5-éthyl-5-méthylhydantoïne, une N,N'-bromochloro-5,5-diméthylhydantoïne ou la 1,3-dibromo-5,5-diméthylhydantoïne, ou un mélange de deux quelconques ou plus desdites hydantoïnes.

46. Composition selon la revendication 45, dans laquelle ladite cire est une cire de paraffine hydrocarbonée.

47. Composition selon la revendication 1, dans laquelle ladite hydantoïne halogénée présente, avant compactage, une taille moyenne de particules inférieure à environ 200 microns, et est la 1,3-dichloro-5,5-diméthylhydantoïne, 1,3-dichloro-5-éthyl-5-méthylhydantoïne, une N,N'-bromochloro-5,5-diméthylhydantoïne ou la 1,3-dibromo-5,5-diméthylhydantoïne, ou un mélange de deux quelconques ou plus desdites hydantoïnes.

48. Composition selon la revendication 47, dans laquelle ladite cire est une cire de paraffine hydrocarbonée.

49. Composition selon la revendication 1, dans laquelle ladite hydantoïne halogénée présente une taille moyenne de particules d'au moins environ 300 microns avant compactage et est la 1,3-dihalo-5,5-diméthylhydantoïne dans laquelle chaque atome d'halogène est, indépendamment, un atome de chlore ou un atome de brome et dans laquelle ladite cire présente, avant compactage, une taille minimale de particules d'au moins environ 10 microns et une taille maximale de particules qui n'est pas supérieure à environ 0,5 mm.

50. Composition selon la revendication 49, dans laquelle ladite cire est une cire de paraffine hydrocarbonée.

51. Procédé de fabrication d'une composition compacte à mémoire de forme selon la revendication 1, ledit procédé comprenant un compactage par pression d'un mélange d'une hydantoïne halogénée poudreuse ou finement divisée et d'une quantité de liant d'une cire de paraffine, dans laquelle ladite cire de paraffine est une cire de paraffine hydrocarbonée et/ou une cire de paraffine chlorée, ladite cire étant compatible avec ladite hydantoïne halogénée.

52. Procédé selon la revendication 51, dans lequel ladite cire présente, avant compactage, une taille minimale de particules d'au moins environ 10 microns.

53. Procédé selon la revendication 51, dans lequel ladite cire présente, avant compactage, une taille maximale de particules qui n'est pas supérieure à environ 0,5 mm.

54. Procédé selon la revendication 51, dans lequel ladite cire, avant compactage, commence à fondre à une température d'au moins environ 50°C.

55. Procédé selon la revendication 51, dans lequel ladite cire présente, avant compactage, une taille de particules minimale d'au moins environ 10 microns et une taille de particules maximale qui n'est pas supérieure à environ 0,5 mm.

56. Procédé selon l'une quelconque des revendications 51 à 55, dans lequel le mélange est réalisé dans un mélangeur par gravité de ladite hydantoïne halogénée poudreuse ou finement divisée et de ladite cire de paraffine de sorte à produire un mélange sensiblement uniforme.

57. Procédé selon l'une quelconque des revendications 51 à 55, dans lequel le mélange est réalisé dans un mélangeur à ruban de ladite hydantoïne halogénée poudreuse ou finement divisée et de ladite cire de paraffine de sorte à produire un mélange sensiblement uniforme.

58. Procédé selon la revendication 51, dans lequel le compactage par pression est réalisé à une pression dans l'intervalle de 1000 à 30000 psi.

59. Utilisation d'un mélange sec dans la production d'une composition compactée par pression à mémoire de forme telle que définie à la revendication 1.

60. Utilisation selon la revendication 59, dans laquelle la quantité de ladite cire est efficace pour réaliser un article à mémoire de forme lorsque ledit mélange est soumis à un compactage par pression.

61. Utilisation selon la revendication 60, dans lequel ledit mélange comprend en outre au moins un excipient ou support.

62. Utilisation selon l'une quelconque des revendications 59 à 61, dans laquelle l'hydantoïne halogénée est la 1,3-dihalo-5,5-dialkylhydantoïne.

63. Utilisation selon la revendication 59, dans laquelle ladite hydantoïne halogénée est la 1,3-dibromo-5,5-diméthylhydantoïne, dans laquelle la cire est une cire de paraffine hydrocarbonée et dans laquelle la quantité de cire est dans l'intervalle de 0,5 à 10 % en poids, de préférence de 1 à 5 % en poids du poids total de la 1,3-dibromo-5,5-diméthylhydantoïne et de la cire.

64. Utilisation selon la revendication 63, dans laquelle ladite cire de paraffine hydrocarbonée, avant compactage, commence à fondre à une température d'au moins environ 50°C.

65. Utilisation selon la revendication 63, dans laquelle la dite cire de paraffine hydrocarbonée présente, avant compactage, une taille minimale de particules d'au moins environ 10 microns.

66. Utilisation selon la revendication 63, dans laquelle ladite cire de paraffine hydrocarbonée présente, avant compactage, une taille maximale de particules qui n'est pas supérieure à environ 0,5 mm.

67. Utilisation selon la revendication 63, dans laquelle ladite cire de paraffine hydrocarbonée, avant compactage, commence à fondre à une température d'au moins environ 50°C, présente une taille minimale de particules d'au moins environ 10 microns, et présente une taille maximale de particules qui n'est pas supérieure à environ 0,5 mm.

68. Forme compacte ou forme réalisée par compactage par pression d'un mélange selon l'une quelconque des revendications 63 à 67.

69. Composition compacte selon la revendication 1, étant un granulé, une capsule, un comprimé, une briquette ou un palet réalisé par compactage par pression d'un mélange sec constitué essentiellement par (i) des particules solides de 1,3-dibromo-5,5-diméthylhydantoïne, et (ii) dune cire de paraffine hydrocarbonée en une quantité dans l'intervalle de 0,5 à 10 % en poids du poids total de la 1,3-dibromo-5,5-diméthylhydantoïne et de la cire.

70. Composition compacte selon la revendication 69, dans laquelle ladite composition compacte est un comprimé, dans laquelle lesdites particules solides de 1,3-dibromo-5,5-diméthylhydantoïne présentent une taille moyenne de particules dans l'intervalle de 125 à 300 microns, et dans laquelle ladite quantité est dans l'intervalle de 1 à 5 % en poids du poids total de la 1,3-dibromo-5,5-diméthylhydantoïne et de la cire.

71. Composition selon les revendications 69 ou 70, dans laquelle ladite cire de paraffine hydrocarbonée, avant compactage, commence à fondre à une température d'au moins environ 50°C, présente une taille minimale de particules d'au moins environ 10 microns, et présente une taille maximale de particules qui n'est pas supérieure à environ 0,5 mm.
